Europäisches Patentamt

(19) European Patent Office

Office européen des brevets

(11) Publication number: **0 168 174**
**A1**

## (12) EUROPEAN PATENT APPLICATION

(21) Application number: **85304118.4**

(22) Date of filing: **11.06.85**

(51) Int. Cl.⁴: **A 61 M 25/02**
**A 61 F 13/02, A 61 L 15/06**

(30) Priority: **13.06.84 US 620173**

(43) Date of publication of application:
**15.01.86 Bulletin 86/3**

(84) Designated Contracting States:
**AT BE CH DE FR GB IT LI NL SE**

(71) Applicant: **Smith & Nephew Inc.**
**2000 South Beltline Boulevard**
**Columbia South Carolina 29205(US)**

(72) Inventor: **Sanner, Alf Thomas**
**4 Coldbranch Court**
**Columbia South Carolina(US)**

(74) Representative: **Cole, William Gwyn**
**Corporate Patents Department Smith and Nephew**
**Research Limited Gilston Park**
**Harlow Essex CM20 2RQ(GB)**

(54) **Film dressing.**

(57) A water vapor permeable disposable dressing comprises a first adhesive contact layer 20 covering an inner surface of a thin film backing 18. A release layer 40 covering the first adhesive contact layer 20 is removed prior to application of the dressing, and the film backing 18 preferably has an edge portion 56 free of adhesive 20 to aid in the removal of the release layer 40 from the dressing without touch contaminating the underside of the dressing. A relatively rigid carrier section 22 is secured to the outer surface of the film backing 18 by a second adhesive layer 24, and the carrier section 22 includes 2 rigid handles 32 which aid in keeping the dressing extended during application of the dressing to a skin surface and prevents the dressing from curling or folding up. Also disclosed is a method for applying the dressing to a patient such as to cover a wound or to secure an intravenous catheter.

Fig.2

Fig.3

EP 0 168 174 A1

Film Dressing

## Background of the Invention

This invention relates to an improved disposable dressing. More particularly, this invention relates to a water vapor permeable disposable dressing for use on human or animal skin for treating wounds or as intravenous catheter covering.

The basic concept of a dressing for a wound or an intravenous catheter is to protect the affected area from contact with fluids, dirt, bacteria or other contaminants while permitting water capor to exit the skin surface. A threshold or common denominator of almost all such dressings is that the dressing be firmly secured about the affected area. Moreover, if a dressing is used in combination with an intravenous catheter dressing, the dressing should securely hold the catheter in place. Accordingly, over the years the industry has attempted to construct dressings which are water vapor permeable and but impervious to liquids and bacteria and which can be held to the skin surface in a sufficiently firm manner to maintain an intravenous catheter in a stationary position.

In this regard, dressings are commonly constructed from composite layers of film and adhesive materials having relatively high water capor permeability such as those disclosed in U.S. Patent No. 3,645,835, which is incorporated herein by reference. The film layer is frequently transparent for ease of inspection of the area and formed from a synthetic polymeric material having a moisture vapor permeability of at least 300 g./sq. meter/24 hours/40°C/80 percent relative humidity (R.H.).

Evidence from various sources indicate that the average body loss of water through the skin excluding visible sweat is in the region of 250 g./sq. meter/24 hours. Areas such as the palm of the hand and soles of the feet, however, have a high water loss in the region of 500 g./sq. meter/24 hours. It follows that a permeability of at least 300 g./sq. meter/24 hours/40°C/80 percent R.H. is required for most areas but a figure of at least 500 is preferred. The adhesive coating which is applied to these dressings also preferably has a water vapor transmission rate which is sufficient to allow the composite dressing to have a moisture vapor transmission rate of at least $300g/m^2 24$ hrs/100-20% R.H.

To obtain the desired water vapor transmission rate, the film layer of the dressing is often constructed from polyurethane synthetic polymeric materials which have the desired water vapor transmission characteristics and which are less than 50 microns in thickness. Although such a film is flexible and can be applied to the varying contours of a body, the thin film is also relatively limp and unmanageable which creates substantial problems in the application of the dressing to a patient.

In this regard, dressings are commonly manufactured with a release sheet covering the adhesive surface of the dressing, with the release sheet being removed when the dressing is applied to the patient. A significant problem which is frequently encountered during this procedure is that the thin flexible film becomes twisted and turns over itself so that portions of the adhesive surface become stuck together. When this occurs, the dressing becomes difficult to apply to a patient. Accordingly, the dressing must be either discarded, resulting in a substantial loss of time and materials, or an attempt made to untwist the dressing at some risk of contamination to the patient.

A significant advance in the multiple layer dressing industry occurred in the relatively recent past when it was determined that a reinforced sheet could be added to the outer surface of the film to reduce the instances of dressing twisting during application. While such systems have achieved at least a degree of industry recognition, room for significant improvement re-

- 3 -

0168174

mains. In this regard, heretofore known reinforcing sheets which overlay the film have onstructed the view of the clinician and interfered with an accurate placement of the dressing. An exact placement is particularly important in instances in which the dressing serves to secure an intravenous catheter attached to a cannular, and improper placement of the dressing can result in the necessity of removal and replacement of both the catheter and of the dressing. Moreover, prior reinforcing sheets have also been relatively complex and not easily understood or manipulated by the clinician. Furthermore, even in instances of proper placement the manipulation involved in applying the dressing has often resulted in an uneven adherence of the dressing to the skin, causing the edges of the dressing to roll back and eventually dislodge.

Problems suggested in the preceding are not intended to be exhaustive, but rather are among many which may tend to reduce the effectiveness of prior dressings. Other noteworthy problems may also exist, however, those presented above should be sufficient to demonstrate that water vapor permeable disposable dressings appearing in the prior art have not been altogether satisfactory.

## Objects

It, therefore, a general object of the invention to provide a water vapor permeable disposable dressing which will obviate or minimize problems of the type previously described.

It is a particular object of the invention to provide a novel disposable dressing which is water vapor permeable but impervious to liquids, dirt, bacteria, and other such contaminants.

It is another object of the invention to provide a water vapor permeable dressing which resists twisting and which is easily applied by a clinician.

It is yet another object of the invention to provide a novel dressing which provides for exact placement of the dressing over the contact surface.

It is still another object of the invention to provide a novel dressing which is held firmly in place about the contact surface and which resists dislodgement.

It is a further object of the invention to provide a novel water vapor permeable dressing which provides for ready manipulation of a cannular end catheter arrangement.

It is yet a further object of the present invention to provide a novel method for applying a disposable dressing to a patient to protect the area of a wound.

It is still a further object of the present invention to provide a novel method for securing an intravenous catheter and protecting the surrounding area from contamination by means of a disposable dressing.

## Brief Summary of the Invention

One preferred embodiment of the invention which is intended to accomplish at least some of the foregoing objects comprises a water vapor permeable disposable dressing having a thin film backing layer with an inner surface and outer surface. A first adhesive layer is located on the inner surface of the thin film backing layer, and a relatively rigid carrier section is secured to the outer surface of the thin film backing. The carrier section has a first portion overlaying the thin film backing layer

and a second handle portion for facile manipulation of the dressing and for removal of the carrier section from the outer surface
of the thin film backing layer so that the dressing is maintained
in a smooth and even fashion during application of the dressing
to a surface.

In a particularly preferred embodiment, the thin film backing layer is comprised of a thermoplastic polyurethane material,
the carrier section is comprised of polyethylene and secured to
the outer surface of the film backing by means of an adhesive
binder, and the carrier section is formed as a split backing with
a central split and two centrally placed handle tabs extending
outwardly for facile manipulation.

In another preferred embodiment, the present invention comprises a process for applying the above described water vapor
permeable disposable dressing having the steps of grasping a
nonadhesive portion of the thin film backing layer and peeling a
release layer from an adhesive layer covering a second portion of
the backing layer. Upon removal of the release layer, the second
handle portion of the relatively rigid carrier section is grasped
whereby the thin film backing layer is distended and applied at
the desired location on the patient. The relatively rigid
carrier section is then removed from the outer layer of the thin
film backing layer.

In yet another embodiment, the present invention comprises a
process for securing an intravenous catheter which is attached to
a cannular and protecting the surrounding area of the patient
from contamination by applying a water vapor disposable dressing
having a thin film backing layer with an inner surface and an
outer surface, a first adhesive layer located on the inner surface of the thin film backing layer, a release layer overlaying
the adhesive layer, and a relatively rigid carrier section
secured to the outer surface of the thin film backing layer. The carrier section has a first portion overlaying the thin film backing layer and a second handle portion, and the carrier section is shaped to expose a window portion of the thin film backing layer in the central area of the layer. The method of securing the intravenous catheter comprises the steps of grasping a nonadhesive portion of the thin film backing layer and peeling the release layer from the adhesive layer. Upon removal of the release layer, the second handle portion of the relatively rigid carrier section is grasped whereby the thin film backing layer is distended and applied at the desired location on the patient so that the window portion of the backing layer is positioned adjacent the point of connection between the catheter and the cannular. The relatively rigid carrier section is then removed from the outer layer of the thin film backing layer.

In describing the invention, term "dressing" the has been used in particular relation to a covering for an intravenous catheter. It is fully intended, however, that the dressing of the present invention may also be used for a wide variety of purposes such as the treatment of burns, punctures, wounds and the like.

## The Drawings

Other objects and advantages of the present invention will become apparent from the following detailed description of a preferred embodiment taken in conjunction with the accompanying drawings, wherein:

Figure 1 is a schematic view of the dressing of the present invention covering the area around an intravenous catheter in a human arm.

Figure 2 is a plan view of the dressing of the present invention.

Figure 3 is a cross sectional view of the dressing of the present invention separated to show the various courses of the dressing.

Figures 4A-E illustrate steps involved in securing a catheter to a patient by means of the dressing of the present invention.

## Detailed Description

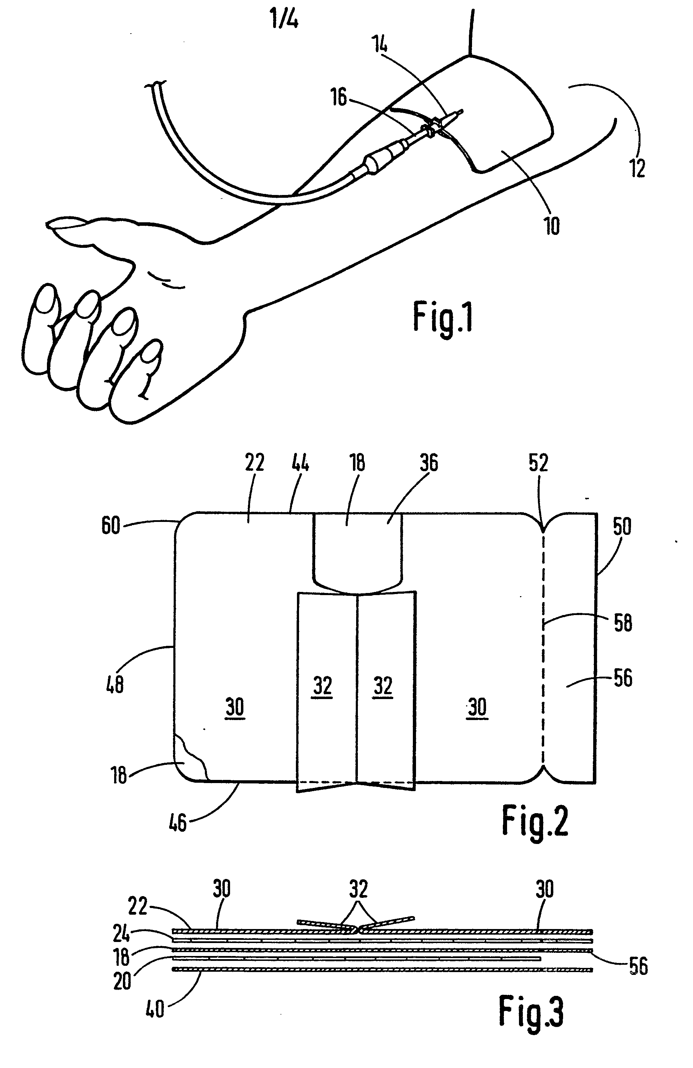

Referring now to the drawings, wherein like numerals indicate like parts, in Figure 1 a schematic view can be seen of a general operative environment of the invention. In this regard, a dressing 10 constructed in accordance to the present invention is applied to a patient's arm 12 in order to secure an intravenous catheter 14 which is connected to cannular 16 and also to protect the surrounding area from contamination.

As shown in Figures 2 and 3, the dressing of the present invention is constructed of multiple layers and comprises a thin film layer 18 overlayed on one side by an adhesive contact layer 20 and on the other side by a relatively rigid carrier section 22. The carrier section 22, which is preferably secured to the film layer 18 by means of an adhesive layer 24, may be formed from material such as polyethylene and is shaped into film overlaying portions 30 and handle portions 32 which are centrally placed in a split back arrangement. Moreover, as seen best in Figure 2, a window 36 is cut from the carrier section at the center of the dressing where the relatively rigid handle portions meet to expose the film layer 18 at a critical juncture where it contacts the catheter. Placement of a window at this location removes all obstructions and assures a good occlusive seal of the dressing around the catheter and protects the intravenous site from migrating bacteria.

The thin film layer 18 is preferably a hydrophylic, polymeric material through which water vapor is capable of diffusing but which is impervious to liquids. Films that may be used in the present invention include the polyurethane films which are described in U.S. Patent No. 3,645,835, British Patent No. 1,280,631, and hydrophilic polyurethane films which are described in U.S. Patent Application Nos. 541,324 and 539,098 and European Patent Application Nos. 91,800 and 84 302 322.7 which are incorporated herein by reference. Generally, this film will have a thickness of between 10 and 50 microns and preferably between 10 and 40 microns, and a vapor transmission rate of between 300 and $5000g/m^2/24$ hrs when not in contact with water (determinable by a method given in European Patent Application No. 91,800 which is incorporated herein by reference). The film layer is preferably continuous in that it contains substantially no perforations or pores (including micropores) in the body contacting portion of the film which extend through the depth of the film and may be formed by extrusion, solvent casting or other known film making methods.

On the inner surface of the thin film layer is a skin-adhesive layer 20. The particular adhesive that is employed may be selected from one of the well known skin contact adhesives such as those disclosed in U.S. Patent No. 3,645,835, British Patent No. 1,280,631, U.S. Patent Application Nos. 541,324 and 539,098 and in European Patent Application Nos. 91,800 and 84 302 322.7.

The adhesive materials of the invention should be nontoxic, nonirritant, conformable, should maintain their properties over a wide range of conditions and time, and should be usable on any part of the body. Continuous adhesives which may be used in the present invention may be formed from polymers containing hydrophilic groups such as hydroxyls, carboxyls, amines, amides, ethers and alkoxys providing that the adhesives are not soluble or highly swollen in water. However, water-soluble or water-swellable polymers can be added to pressure sensitive formulations provided they are compatible and do not cause the adhesives
to be affected by water (for example a polyvinylmethyl ether may
be added to a polyvinylethyl ether adhesive and/or a hydroxy
propyl acrylate homopolymer may be added to a compatible pressure-sensitive adhesive); other water-soluble or water-swellable
polymers which may be used are cellulose esters, polyvinyl alcohol and other hydrophilic materials. Similarly, water-soluble
monomers can be used in water-insensitive copolymers.

Examples of materials which may be used as the continuous
adhesive are blends of vinyl ether or acrylic polymers, with or
without the addition of tackifying resins. Hydroxy acrylate
polymers may also be used in suitable formulations. Preferred
water-impermeable adhesives are polyvinyl ethyl ethers and certain acrylate ester copolymers containing hydrophilic groups.

The adhesive may be applied to the film layer in solution,
aqueous dispersion, as a hot melt, or by a transfer process,
using known techniques, e.g., knife, roller-coating or curtain
coating methods. The level of the adhesive on the film should
not be so greater, however, that the water vapor transmission
characteristics of the film are impeded. Generally, a coating
level of from 15 to 60, more usually 20 to 40g/m$^2$ is sufficient
to obtain adequate skin adhesion but not so great as to interfere
with the water vapor transmission characteristics desired in the
finished dressing. The adhesive mass may be applied directly to
the film or may be applied to a silicone-coated sheet and the
film then brought into contact with the adhesive on the sheet.
The film may be removed from the sheet for subsequent processing,
or the sheet may remain with the film and become the release
layer 50 in the finished dressing.

As shown in Figure 2, the dressing has a top edge 44 and
bottom edge 46 and two opposing extension side edges 48 and 50 and opposing interior side edge 52 formed along the perforation line and the tab 56. The film layer 18 is preferably coated with adhesive layer 20 from the top edge to the bottom edge but not to the right exterior side edge under the handle 56, which is best shown in Figure 3. The interior side edge 52 is formed by a series of perforations 58 which extend through the film. The perforations 58 may also extend partially or completely through the release sheet 40 which preferably covers the entire inner adhesive side of film 18. The perforation runs in a straight line from the top to the bottom edges of the film.

The ratio of the cut areas of the perforation line to the uncut areas of the perforations should be such that the film can be removed from the carrier sheet without tearing the film along the perforation line, but the perforation should be readily broken after the film dressing is applied to the skin of the patient.

The portion of the film between the perforation line 52 or interior side edge of the film dressing and the exterior side edge 50 of the dressing is a handling tab 56 which is free of adhesive. The tab is used to hold the dressing to remove it from the release sheet and to apply the dressing to the patient.

The adhesive 20 is applied to the entire underside of the film up to the perforation line 24 and the beginning of the tab 56. This line will favor slightly the adhesive area of the film so as to assure that the adhesive is completely applied to the patient covering film area. This avoids a nonadhesive edge which could cause the dressing to pull up and dislodge from the patient. The corners of the dressing may be rounded to make them less likely to lift and roll off the patient.

Figures 2 and 3 also illustrate a relatively rigid film carrier section 22 consisting of two separate transparent sheet portions 30 affixed to the back of the film by adhesive layer 24 and each having a centrally positioned handle portion 32. The carrier section is preferably formed from polyethylene which is from 20 to 120 microns in thickness. The rigid carrier section adheres to the film from the top edge 44 to the bottom edge 46 and from the two opposing sides 48 and 50. Each handle portion 32 is formed by extending the length of the overlaying portion 30 widthwise to the middle of the adhesive of the dressing 10 between edges 48 and 52 and folding the polyethylene back to form handles 30 of 2cm to 3cm in width. This system serves to keep the thin polyurethane film layer 18 neatly extended for easy application of the dressing to the patient once the release sheet 40 has been removed and effectively prevents the dressing from folding in on itself or curling up which would result in a complete loss of the dressing. The rigid carrier section is removed easily once the dressing has been applied to the skin by pulling on the handles 32 in opposite directions and away from the middle.

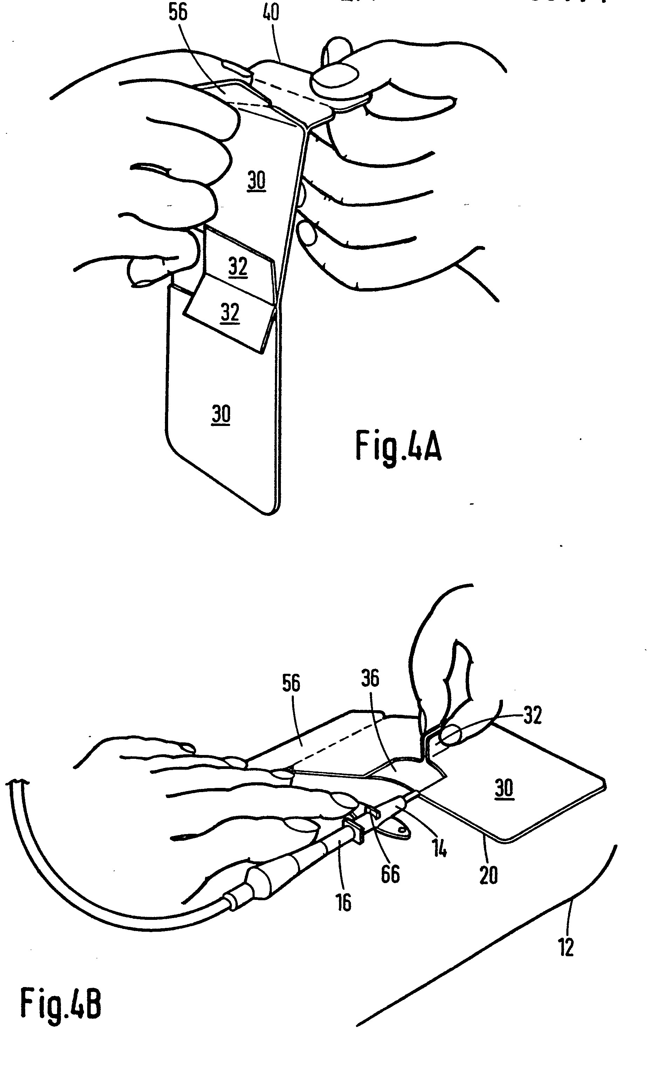

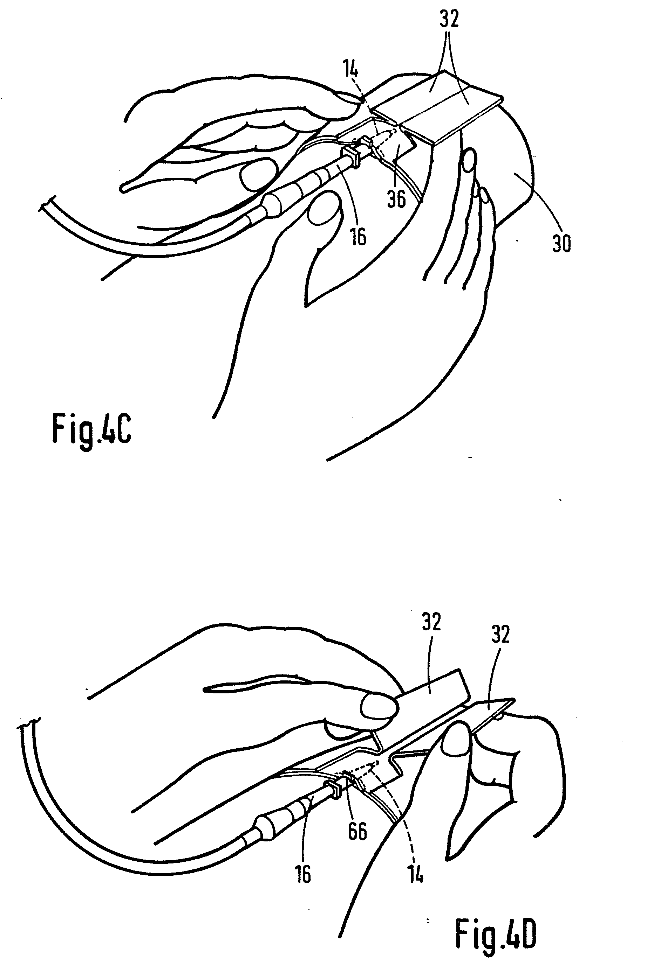

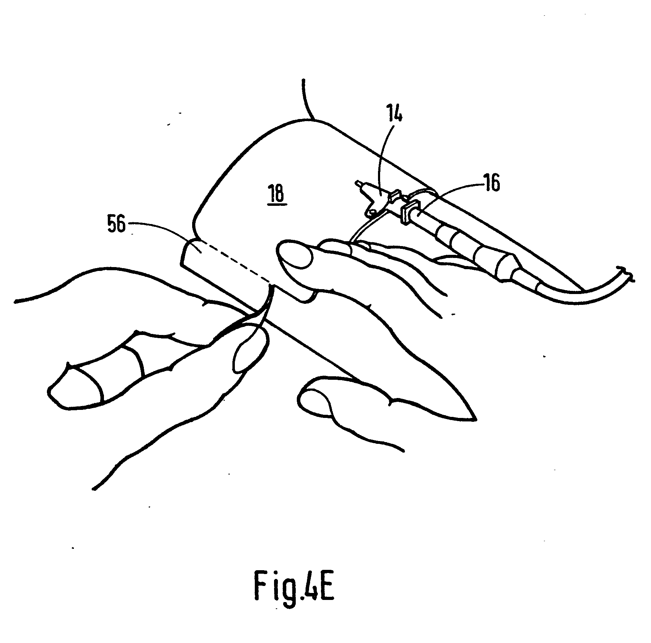

Figures 4 (a) - (e) illustrate the steps by which an intravenous catheter may be held in place and the surrounding area protected from contamination by the dressing of the present invention. After insertion of the catheter into the patient's arm, the release sheet 40 covering the adhesive contact layer 20 is readily removed from the dressing by bending the uncoated handle tab 56 away from the release layer 40 and and pealing the release layer from the dressing as shown in Figure 4a.

After the release layer has been removed, the dressing is applied to the area surrounding the catheter insertion by clasping the centrally placed handle tabs 32 together as shown in Figure 4b. In this position, the carrier section maintains the thin film layer and the adhesive layer covering the inner surface of the thin film in a smooth and untwisted orientation which can be easily manipulated. Moreover, the window 36 in the carrier section provides the clinician with an unobstructed view of the edge 66 of catheter 14 for exact placement of the dressing. Placement of the dressing at this location allows cannular 16 to be detached from the catheter 14 for any purpose without removal of either the catheter or the dressing.

As shown in Figure 4c, after the dressing is applied to the desired location the film may be smoothed by hand or otherwise contoured to the part of the body in which the dressing has been placed. To remove the carrier section from the dressing, the handle tabs 32 are grasped as shown in Figure 4d and pulled in opposite directions towards the side edges of the dressing exposing the outer surface of thin film layer 18. At this point, the non-adhesive covered tab 56 may be removed as shown in Figure 4a, if desired, by tearing along the perforated line and the film layer smoothed by hand to ensure a secure and even application. In the working position shown in Figure 1 the dressing generally will not be removed until the removal or replacement of the catheter.

Having described in detail a preferred embodiment of the invention and before continuing with the claim portion of the specification, it may be useful to briefly set forth some of the major advantages of the invention.

### Summary of Major Advantages of the Invention

In describing a water vapor permeable disposable dressing in accordance with a preferred embodiment of theinvention those skilled in the art will renognize several advantages which singularly distinguish the subject invention from the heretofore known prior art.

A particular advantage of the subject invention is the provision of a water vapor permeable disposable dressing which may be facile manipulated and contoured to various skin surfaces. In this connection, in prior known dressings it was often necessary to prematurely discard dressings before application to a patient because of twisting or curling up. This twisting of dressings resulted in a substantial loss of materials and clinicians time as well as extending the time required to properly treat the patient.

Another significant advantage of the subject invention is the provision of a window in the central portion of the carrier section to provide an unobstructed view to a clinician of the critical area of catheter insertion. Placement of the dressing at this location allows a cannular to be disconnected from a catheter without changing the dressing, which would otherwise have to be changed if the dressing were improperly placed.

In describing the invention, reference has been made to a preferred embodiment. Those skilled in the art, however, and familiar with the disclosure of the subject invention may recognize additions, deletions, substitutions, modifications and/or other changes which will fall within the perview of the invention as defined in the following claims.

CLAIMS

1.    A water-vapour permeable disposable dressing comprising a film backing layer having an inner surface and an outer surface, a first adhesive layer located on the inner surface of the film backing layer and a carrier section secured to the outer surface of the film backing layer characterised in that the carrier section has a first portion overlaying the film backing layer and a second handle portion for facile manipulation of the dressing and for removal of the carrier section from the outer surface of the film backing layer so that the dressing is maintained in a relatively smooth and even fashion during application to a surface.

2.    A dressing as claimed in claim 1 in which the carrier section has a split backing with a central split and two centrally placed handle tabs extending from the overlying portions of the carrier section.

3.    A dressing as claimed in claim 2 in which the split carrier section is shaped to expose a portion of the film backing layer in the central area of the film backing layer and along the meeting edges of the overlying portions of the carrier section.

4.     A dressing as claimed in any one of claims 1 to 3
in which the carrier section is releasably secured to
the outer surface of the film backing layer by means
of a second adhesive layer.

5.     A dressing as claimed in any one of claims 1 to 4
in which the first and second adhesive layers are
comprised of polyvinyl ethyl ethers or an acrylic ester
copolymer.

6.     A dressing as claimed in any one of claims 1 to 5
in which the film backing layer comprises a thermoplastic
polyurethane film.

7.     A dressing as claimed an any one of claims 1 to 6
in which the film backing layer has a thickness of from
10 to 50 µm.

8.     A dressing as claimed in any one of claims 1 to 7
in which the carrier section is comprised of polyethylene.

9.     A dressing as claimed in any one of claims 1 to 8
in which a non-adhesive portion of the film backing layer
is attached along a perforation to an edge of the adhesive
coated film.

Fig.1

Fig.2

Fig.3

Fig.4A

Fig.4B

Fig.4C

Fig.4D

Fig.4E

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-3 863 631 (B.E. BALDWIN) <br> * Column 3, lines 1-64; column 5, lines 3-24; figures 1,11,12 * | 1-4,8 | A 61 M 25/02 <br> A 61 L 15/06 <br> A 61 F 13/02 |
| X | EP-A-0 081 990 (JOHNSON & JOHNSON) <br> * Whole document * | 1-7 | |
| X | EP-A-0 066 899 (J.D. MUCHLIN) <br> * Page 8, line 16 - page 11, line 19; figures 1,3 * | 1,4-8 | |
| A | | 3,9 | |
| X | GB-A-2 120 104 (THE KENDALL CO.) <br> * Whole document * | 1,4-9 | **TECHNICAL FIELDS SEARCHED (Int. Cl.4)** |
| A | DE-A-2 947 275 (MO OCH DOMSJÖ) <br><br> * Page 4, line 36 - page 5, line 38; figure 1 * | 1,2,4, 8 | A 61 M <br> A 61 F |
| A | GB-A-2 035 096 (L.I.C.) <br> * Figures 1-3; abstract * | 2,3,9 | |
| A | US-A-3 826 254 (E.K. MELLOR) <br><br> * Column 2, lines 9-54; figures 1-4 * | 1,3,4, 6 | |

--- -/-

The present search report has been drawn up for all claims

| Place of search | Date of completion of the search | Examiner |
|---|---|---|
| THE HAGUE | 20-09-1985 | WOLF C.H.S. |

| | DOCUMENTS CONSIDERED TO BE RELEVANT | | Page 2 |
|---|---|---|---|

| Category | Citation of document with indication, where appropriate, of relevant passages | Relevant to claim | CLASSIFICATION OF THE APPLICATION (Int. Cl.4) |
|---|---|---|---|
| A | US-A-3 918 446 (P.M. BUTTARAVOLI) * Column 4, lines 26-30; figures 1-3, 13,14 * | 1-4 | |
| P,A | EP-A-0 117 632 (JOHNSON & JOHNSON) * Abstract * | 3 | |
| A | US-A-2 082 219 (W.M. SCHOLL) * Figure 1 * | 3 | |
| A | US-A-3 025 957 (T.H. WALL) * Column 2, lines 38-64; figures 1,2,5 * | 8 | |
| | | | TECHNICAL FIELDS SEARCHED (Int. Cl.4) |

The present search report has been drawn up for all claims

| Place of search | Date of completion of the search | Examiner |
|---|---|---|
| THE HAGUE | 20-09-1985 | WOLF C.H.S. |